(19) Europäisches Patentamt
European Patent Office
Office européen des brevets

(11) Publication number: 0 171 811
A2

(12) # EUROPEAN PATENT APPLICATION

(21) Application number: 85110228.5

(22) Date of filing: 14.08.85

(51) Int. Cl.[4]: C 07 D 498/04
//(C07D498/04, 263:00, 263:00)

(30) Priority: 16.08.84 US 641242

(43) Date of publication of application:
19.02.86 Bulletin 86/8

(84) Designated Contracting States:
AT BE CH DE FR GB IT LI LU NL SE

(71) Applicant: Ashland Oil, Inc.
1401 Winchester Avenue
Ashland Kentucky 41101(US)

(72) Inventor: Goel, Anil B.
373 Eastworth Court
Worthington Ohio 43085(US)

(72) Inventor: Richards, Harvey J.
2210 Bristol Road
Columbus Ohio 43221(US)

(74) Representative: Vossius Vossius Tauchner Heunemann Rauh
Siebertstrasse 4 P.O. Box 86 07 67
D-8000 München 86(DE)

(54) **Bicyclic amide acetal production.**

(57) An improved process for the manufacture of bicyclic amide acetals by the reaction of diethanol amine with a nitrile in the presence of an alkali metal or alkaline earth metal catalyst and continuous removal of the ammonia by-product from the reaction mixture as it forms is described.

SSIUS · VOSSIUS · TAUCHNER · HEUNEMANN · RAUH
PATENTANWÄLTE
SIEBERTSTRASSE 4 8000 MÜNCHEN 86 PHONE: (089) 474075
CABLE: BENZOLPATENT MÜNCHEN · TELEX 5-29 453 VOPAT D

0171811

Our Ref.: T 909 EP
Case: 4722
Ashland Oil, Inc.

August 14, 1985

# BICYCLIC AMIDE ACETAL PRODUCTION

This invention relates to an improved method for the production of bicyclic amide acetals and more particularly pertains to an improved process for preparing bicyclic amide acetals in high yields which involves the reaction of alkyl nitriles with diethanol amine in the presence of a catalyst in which the nitrile is fed to the diethanol amine and the ammonia evolved in the reaction is removed from the reaction as it forms by purging with nitrogen or other inert gas.

The synthesis of bicyclic amide acetals by reaction of diethanol amine with alkyl nitriles has been reported in Angew. Chem., 85, 1055 (1973). The yields reported were low (30-40%) and although few details were given as to the preparation, this reported reaction required long reaction times (20-40 hours) and relatively high temperatures (140°C.) to produce the relatively low yields. Because of the reported long reaction times and poor yields, the prior art process is not attractive from a commercial standpoint.

We have discovered an improved method for synthesizing bicyclic amide acetals comprising reacting diethanol amine with an alkyl nitrile in the presence of a catalyst such as sodium in which the reaction temperature is maintained at about 100°C., the alkyl nitrile is fed continuously to the reactor over the course of the reaction, the ammonia formed in the reaction is removed by sparging with dry inert gas such as nitrogen, argon, etc., during the reaction, and the bicyclic amide acetal is removed from the reactor before it reaches about 50% concentration based on the weight of the reaction mixture. Our process produces bicyclic amide acetal in yields of 60% or greater and in relatively short reaction times.

Our process can be illustrated by the following equation:

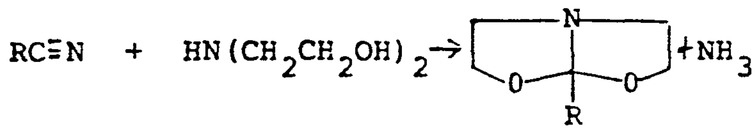

(I)

Wherein R represents a hydrocarbon group having from 1 to 20 carbon atoms, and preferably an alkyl, aralkyl or alicyclic group. Preferably, the alkyl group has from 1 to 12 carbon atoms e.g.methyl-, ethyl- or propyl-, the aralkyl group has from 7 to 14 carbon atoms, and the alicyclic group has from 5 to 12 carbon atoms. In our process, the reaction temperature is maintained in the range of from about 80°C. to 120°C. and preferably about 100°C., an inert gas such as nitrogen is bubbled continuously through the reaction mixture to sweep out the ammonia as it forms and the alkyl nitrile is introduced into the reactor continuously (can be regulated by the temperature controller). Suitable catalysts include alkali and alkaline earth metals and their salts. Catalysts can be used in amounts of 5 mole percent or less. In our process, it is desirable to stop the reaction after about 40-50% of bicyclic amide acetal has formed in the reaction mixture (usually 5-8 hours from the start of the reaction) and to remove the bicyclic amide acetal from the reaction mixture at reduced pressure. The remaining diethanol amine in the reactor is then further reacted with the alkyl nitrile in the usual manner. No additional catalyst is needed for the second stage of the reaction. By operating in this manner, yields in the order of 70% of the desired bicyclic amide acetal can be obtained in about 18 hours. Alternatively, after the first removal of the bicyclic amide acetal, additional diethanol amine can be introduced to the reactor along with alkyl nitrile,

thus bringing the volume of the reaction mixture to the original level and thus a semi-continuous process can be used.

The catalyst used in our process is preferably an alkali metal.

The reaction temperature employed in our process should be in the range of from 80°C. to 120°C. and preferably from 90°C. to 105°C.

Our process is preferably carried out at or near atmospheric pressure although pressures in the range of from 1 to 5 atmospheres can be employed.

The bicyclic amide acetals produced by the process of this invention react rapidly with water and are useful for removing small traces of water from various organic materials such as monomers and polymers.

The process of our invention is further illustrated in the following examples in which the amounts of ingredients are expressed in parts by weight unless otherwise indicated.

## EXAMPLE 1

The bicyclic amide acetal, 1-aza-5-methyl-4, 6-dioxa bicyclo [3.3.0] octane (Formula I in the foregoing equation in which R is methyl) was prepared by adding 1577.3g (15.0 mols) of diethanol amine in a 3 liter 3 necked flask reactor equipped with magnetic stirring bar, thermometer with a temperature controller unit, dry nitrogen sparge tube, dropping funnel and a distillation fractionating head. Sodium metal (6.9g, 0.3 m mol) was added and was allowed to react with the diethanol amine at about 90°C. After the sodium had reacted completely, acetonitrile was added to the reactor continuously at such a rate as to maintain a vigorous reflux at about 100°C. The reaction was carried out in this manner for 6 hours during which about 700g of acetonitrile was added to the reactor.

The ammonia which was formed as a by-product during the entire course of the reaction was vigorously removed by nitrogen sparge. Excess acetonitrile was first removed by distillation at 30-50 mm. Hg (3999-6665 Pa) followed by distillation of the product at 72°C. and 15 mm.Hg. (1999,5 Pa). A total of 813.5 g. of the bicyclic amide acetal was collected and was found to be about 100% pure by GLC analysis. GLC analysis of the reaction residue showed about 7% to be unrecovered bicyclic amide acetal and the remainder to be unreacted diethanol amine. This reaction residue was again treated with acetonitrile at 100°C. for 6 hours in the above-described manner followed again by product isolation and an additional 388.3g of the desired bicyclic amide acetal was obtained. Thus, in 18 hours a yield of 1357.1g of the bicyclic amide acetal product was obtained which is about a 70% yield.

## EXAMPLE 2

This experiment was carried out to demonstrate that a reasonably high yield of the bicyclic amide acetal can be obtained by carrying out the reaction continuously for a longer time at 100°C. but that a slightly lower (about 10%) yield is obtained when the product is not separated as in Example 1.

Diethanol amine (107.3g, 1.02 moles) containing 2 mole percent sodium metal was placed in a 250 ml, 3 necked flask fitted with magnetic stirrer, thermometer with a temperature controller, dry nitrogen sparge, dropping funnel and condenser. Acetonitrile (60.4g, 1.5 moles) was added dropwise over a four hour period to the stirred reaction mixture while continuous sparging and a reaction temperature of about 88°C. were maintained. The reaction temperature was raised to about 100°C. where it was kept for 21 hours. The resulting reaction mixture was analyzed by GLC and was found to have a diethanol amine conversion of 68% with a 90% selectivity to the bicyclic amide acetal which was a 61% overall yield of the desired product.

We Claim:

1. The process for preparing a bicyclic amide acetal having the formula

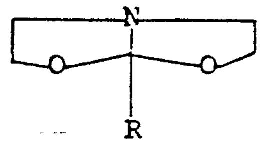

comprising contacting a mixture of diethanolamine and a nitrile having the formula $R-C\equiv N$ wherein R represents an alkyl, aralkyl or alicyclic group with a catalyst composed of an alkali metal or an alkaline earth metal or their salts /at a temperature in the range of from 80° to 120°C. and continuously removing the by-product ammonia from the reaction mixture as it forms.

2. The process of Claim 1 wherein the alkyl group has from 1 to 12 carbon atoms, the aralkyl group has from 7 to 14 carbon atoms and the alicyclic group has from 5 to 12 carbon atoms.

3. The process of Claim 2 wherein R is an alkyl group.

4. The process of Claim 3 wherein R is a methyl group.

5. The process of Claim 4 wherein the catalyst is an alkali metal.

6. The process of Claim 5 wherein the catalyst is sodium.

7. The process of Claim 3 wherein the ammonia is removed by sparging the reaction mixture with an inert gas.

8. The process of Claim 7 wherein the inert gas is nitrogen.